# EUROPÄISCHE PATENTSCHRIFT

(11) **EP 1 202 713 B1**
(45) Veröffentlichungstag und Bekanntmachung des Hinweises auf die Patenterteilung: **18.08.2004**
(21) Anmeldenummer: 00951321.9
(22) Anmeldetag: 30.06.2000
(51) Int. Cl.: A61K 9/107, A61P 27/04

(54) **TRÄNENERSATZMITTEL**
ARTIFICIAL TEAR REPLACEMENT SOLUTION
LARMES ARTIFICIELLES

(30) Priorität: 14.08.1999 DE 19938668
(43) Veröffentlichungstag der Anmeldung: 08.05.2002
(73) Patentinhaber: BAUSCH & LOMB INCORPORATED, Rochester, New York 14604-2701 (US)
(72) Erfinder: MENZ, Dirk-Henning, D-86420 Diedorf (DE); DRESP, Joachim, D-81825 München (DE); WINTER, Martin, D-28717 Bremen (DE)
(74) Vertreter: Maiwald Patentanwalts GmbH
(86) Internationale Anmeldenummer: PCT/EP2000/006109
(87) Internationale Veröffentlichungsnummer: WO 2001/012159

(56) Entgegenhaltungen:
- EP-A- 0 112 658
- EP-A- 0 288 659
- WO-A-95/09606

## Beschreibung

Die Erfindung betrifft die Verwendung eines Gels welches mindestens ein wasserlösliches Fluortensid, Wasser und eine unpolare Komponente, ausgewählt aus teilfluorierten Fluorcarbonverbindungen der allgemeinen Formel R_{F}R_{H} oder R_{F}R_{H}R_{F}, sowie Fluorcarbon-Oligomeren des Typs (R_{F})ₓR_{H}, oder aus einer Mischung hiervon, oder Silikonöl, enthält zur Herstellung eines Tränenersatzmittels zur äußeren Behandlung des Auges.

Tränenersatzmittel finden Verwendung bei der Behandlung von Augenerkrankungen, wie beispielsweise der häufig auftretenden Keratokunjunktivitis sicca (sogenanntes "trockenes Auge"). Ein trockenes Auge kann durch verschiedene Ursachen entstehen. Die häufigsten Ursachen sind u.a. nachlassende Tränenproduktion im Alter, rheumatische oder internistische Erkrankungen, (wie z.B. Polyarthritis, Diabetes und Schilddrüsenkrankheit), Erkrankungen, bei denen Antikörper gegen körpereigene Stoffe gebildet werden (Morbus Sjögren, Lupus erythematodes, Sklerodermie), Hautkrankheiten, Hormonunutellungen, Nervenlähmungen (wie z.B. nach einem Schlaganfall, Lidfehlstellungen, Tränendrüsenentrindungen) Einnahme von bestimmten Medikamenten (wie z.B. β-Blocker, Anti-Baby-Pille, Schlaf- und Beruhigungsmittel), Mangelernährung, klimatische Einflüsse (heiße, trockene Umgebungsluft, Jahreszeit, Klimaanlagen), Umweltbelastungen (Ozon, Stäube, Lösungsmitteldämpfe etc.), Bildschirmarbeiten und chronischer Gebrauch von gefäßzusammenziehenden Augentropfen (sog. Weißmacher). Weiterhin werden derartige Augenerkrankungen mit geringerer Häufigkeit auch durch Autoimmunerkrankungen, Erkrankungen des hämatopoetischen Systems, lokalen Augenerkrankungen wie Entzündungen und Verletzungen der Tränendrüsen oder angeborenen Erkrankungen verursacht. Die dadurch hervorgerufenen Dysfunktionen stören oder verhindern den Aufbau eines normalen Tränenfilms, der in seiner nattitrlichen Ausbildung einen äußerst komplizierten Aufbau besitzt. Im wesentlichen sind drei Komponenten am Aufbau des Tranenfilms beteiligt:

Eine innere Muzinschicht überzieht die Epitheloberfläche, es folgen eine mittlere, wässrige Schicht und eine dünne, äußere Lipidschicht. Die Muzinschicht dient dabei als adhäsive Komponente zur Benetzung der Hornhaut. Die wässrige Komponente befeuchtet die Hornhaut und übt eine reinigende und schützende Funktion aus. Die Lipidkomponente verhindert die Verdunstung der wässrigen Komponente und beugt einem zu schnellen Ablaufen des Tränenfilms vor.

Nur ein intakter Tränenfilm kann auf Dauer die volle Funktionalität der Augenoberfläche gewährleisten, wobei neben den genannten Wirkungen insbesondere auch reibungsmindemde und antibakterielle Eigenschaften und die Versorgung der Hornhaut mit Sauerstoff von Bedeutung sind. Die oben genannten Komponenten der Träne werden ständig produziert. Der Aufbau eines dünnen Tränenfilms über der Hornhaut erfolgt spontan bei jedem Lidschlag, indem durch die Abwärtsbewegung des Oberlides die äußere Lipidschicht des Tränenfilms zwischen den Lidrändern zusammengedrückt wird, wobei die wässrige Schicht im wesentlichen an ihrem Platz verbleibt. Sobald ein Teil der Produktion der Tränenkomponenten unterbrochen oder gestört ist oder der durch den Lidschlag erfolgende Tränenfilmaufbau mechanisch gestört ist, resultieren entsprechende Beschwerden, wie z. B. der sogenannte Sandkorneffekt bis hin zu massiven Sehstörungen, welche im Extremfall durch irreversible Schädigungen der Hornhaut zur Erblindung führen können.

Entsprechend der Vielfalt möglicher Ursachen des "trockenen Auges" und der komplexen Aufgabe des Tränenfilms sind aus dem Stand der Technik eine Vielzahl von Behandlungsmitteln bekannt. Beispielhaft seien hier die Druckschriften EP 698 388, DE 195 11 322, DE 43 03 818, EP 801 948, WO 97/45102, WO 96/33695 genannt.

In den oben genannten Druckschriften sind im wesentlichen Behandlungsmittel beschrieben, welche durch Eingabe in den Bindehautsack eine oder mehrere fehlende Komponenten des natürlichen Tränenfilms ersetzen. Der Ersatz erfolgt hierbei durch Stoffe, welche bei entsprechender Verweilzeit schutz- und reibungsmindernde Funktionen übernehmen und welche die gleichen oder zumindest ähnliche Eigenschaften wie die zu substituierenden Komponenten besitzen. Bei dem im Handel unter dem Markennamen "Liposic" erhältlichen Behandlungsmittel wurde beispielsweise versucht, alle natürlichen Tränenbestandteile in einer sogenannten dreidimensionalen Träne nachzubilden.

Die aus dem Stand der Technik bekannten flüssigen Behandlungsmittel weisen jedoch alle den Nachteil auf, daß sie eine relativ kurze Verweilzeit auf der Hornhaut haben. Dadurch bedingt muß das Behandlungsmittel in regelmäßigen Abständen in das Auge eingebracht werden, was für den Patienten sehr umständlich und unangenehm sein kann. Bei den bekannten gelförmigen Behandlungsmitteln wird dieser Nachteil zumindest teilweise vermieden. Bei gelförmigen Behandlungsmitteln erweist es sich jedoch als schwierig, die Hornhaut ausreichend mit Sauerstoff zu versorgen.

In den Druckschriften EP 089 815 und EP 112 658 werden ophthalmologische Behandlungsmittel zum Schmieren und zum Schutz der Augenoberfläche vorgeschlagen, welche ein Perfluorcarbon oder ein substituiertes Derivat hiervon enthalten. Als wesentliche Vorteile gegenüber den herkömmlichen Behandlungsmitteln wird dabei die Unmischbarkeit mit Wasser und die hohe Gaslösekapazität, insbesondere für Sauerstoff, genannt.

Aufgrund der von Fluorcarbonen bekannten hohen Sauerstofflösekapazität soll durch die dort beschriebenen Behandlungmittel eine ausreichende Sauerstoffversorgung der Hornhaut gewährleistet sein. Darüberhinaus soll aufgrund der hohen Dichte der Perfluorcarbone eine lange Verweilzeit auf der Hornhaut ermöglicht werden, weil die Verbindungen sich wegen ihres hohen spezifischen Gewichts anreichern sollen. Wegen der Unlöslichkeit der Behandlungsmittel in Wasser soll überdies auf Konservierungsmittel verzichtet werden können.

Bei diesen perfluorcarbonhaltigen Behandlungmitteln ist jedoch nachteilig, daß eine intakte Muzinschicht und die ausreichende Sekretion von Lipiden gewährleistet sein muß, um eine ausreichende Funktionalität des Mittels auf der Hornhaut zu ermöglichen. Weitere Nachteile ergeben sich aus der Beeinträchtigung des Visus durch Schlierenbildung sowie der Gefahr von Verstopfungen der Tränenabflußkanäle, hervorgerufen durch die Unmischbarkeit der Perfluorcarbone mit Wasser. Die Unmischbarkeit von Perfluorcarbon und Wasser sowie die große Grenzflächenspannung zwischen wässrigen und perfluorcarbonhaltigen Bereichen führt darüberhinaus zur Entstehung von Diffusionsbarrieren, wodurch eine ausreichende Versorgung der Hornhaut verhindert wird.

Aus dem Stand der Technik sind weiterhin verschiedene Formen von Fluorgelen bekannt, die auch für medizinische Anwendungen vorgeschlagen wurden. Aus der US 5,573,757 und der EP 340 079 sowie der WO 97/03644 sind Polyaphrongele bekannt, deren Struktur durch Fluortenside stabilisiert ist. Die Struktur und die Eigenschaften von Polyaphrongelen sind beispielsweise in Kapitel 8 von "Foams and biliquid Foams-Aphrons", F. Sebba, John Wiley 1987, beschrieben.

Fluorgele als solche weisen im allgemeinen eine ausgeprägte Viskoelastizität auf. Aus diesem Grunde eignen sich diese Stoffe nicht für eine extraokuläre Behandlung von Augenerkrankungen, da es wegen der nicht ausgeprägten Eigenschaft zur Filmbildung nicht möglich ist, eine gleichmäßige Verteilung und Benetzung der Hornhaut zu erreichen.

Es ist daher die Aufgabe der vorliegenden Erfindung ein langzeitverträgliches Tränenersatzmittel zur Verfügung zu stellen, welches eine gleichmäßige Benetzung der Hornhaut ermöglicht, eine lange Verweildauer auf der Augenoberfläche aufweist und die Versorgung der Hornhaut mit Sauerstoff und wasserlöslichen Nährstoffen gewährleistet.

Gelöst wird diese Aufgabe mit den kennzeichnenden Merkmalen von Anspruch 1. Vorteilhafte Ausführungsbeispiele sind den Unteransprüchen zu entnehmen.

Eine der wesentlichen Grundlagen der vorliegenden Erfindung bildet die Erkenntnis, daß eine Präparation, welche Wasser, eine unpolare Komponente und ein wasserlösliches Fluortensid enthält, glatte, hydrophobe Oberflächen gleichmäßig benetzt, sobald die Präparation in flüssiger Form vorliegt. Der Mechanismus der die gleichmäßige Verteilung der flüssigen Präparation als dünnen Film auf der glatten Oberfläche bewirkt, ist die Spreitung der unmischbaren Komponenten der erfindungsgemäßen Präparation aufeinander bzw. auf der hydrophilen oder hydrophoben Oberfläche.

Bei den erfindungsgemäßen Tränenersatzmitteln ist dadurch eine Filmbildung einer wasserhaltigen Präparation auf der hydrophoben Hornhaut ermöglicht. Die Ausbildung des dünnen Films auf der Hornhaut erfolgt dabei durch Lidschlag, wie bei der natürlichen Tränenbildung. Hierbei wird insbesondere die durch die Struktur der Fluortenside gegebene Besonderheit der Kombination hydrophiler, hydrophober und fluorophiler Eigenschaften für die Benetzung der Hornhautoberfläche ausgenutzt. Durch Einhaltung bestimmter Konzentrationen der Fluortenside können darüberhinaus die Oberflächen- und Grenzflächenspannungen der erfindungsgemäßen Komponenten so abgestimmt werden, daß eine optimale Filmbildung und eine ausreichende Versorgung der Hornhaut mit Sauerstoff ermöglicht ist.

Die Ausbildung von dünnen Filmen auf der Hornhautoberfläche führt zu hohem Rückhaltevermögen für die Präparationen und gleichzeitig dazu, daß die Tränensekretion, Schleimproduktion und Sekretion der Lidranddrüsen nicht behindert wird. Außerdem verhindern die entstehenden Filme ein Verkleben des Lids. Außerdem ermöglichen die besonderen Oberflächeneigenschaften der Präparate, daß diese am Ende ihrer Verweilzeit die natürlichen Tränenabflußwege im Auge passieren, ohne sie zu verstopfen. Auf diese Weise können die erfindungsgemäßen Präparationen reibungsmindernde und benetzende Funktionen des natürlichen Tränenfilms übernehmen, ohne unerwünschte Nebenwirkungen, wie z.B. verstopfte Tränenabflußwege, hervorzurufen.

Durch die Kombination der Komponenten des erfindungsgemäßen Tränenersatzmittels ist es ermöglicht, die Vorteile der einzelnen Komponenten zu nutzen ohne Nachteile in Kauf nehmen zu müssen.

Die benetzenden Eigenschaften von Tensiden sind weithin bekannt und finden in vielen technischen Prozessen Verwendung. Allerdings sind gerade diese an sich vorteilhaften Eigenschaften Ursache für ihre schlechte Bioverträglichkeit, welche sich insbesondere durch Störungen in Zellmembranen bemerkbar macht. Daher ist die Verwendung von tensidhaltigen Stoffen für medizinische Anwendungen wegen ihres toxischen Verhaltens stark eingeschränkt. So sind beispielsweise Unverträglichkeiten von sauerstofftransportierenden Emulsionen auf die Wirkung der enthaltenen Tenside zurückzuführen. Insbesondere erscheint die Verwendung von tensidhaltigen Substanzen als Tränenersatzmittel ausgeschlossen, weil die Funktion des natürlichen Tränenfilms entscheidend durch die oberflächenmodifizierenden Eigenschaften der Lipide bewirkt ist und diese besonders empfindlich auf Zusätze anderer oberflächenaktiver Substanzen reagieren.

Überraschenderweise gelang es dennoch, durch die Kombination der Komponenten des erfindungsgemäßen Tränenersatzmittels die oberflächenaktive Wirkung von Tensiden nutzbar zu machen, ohne daß die Funktion der im natürlichen Tränenfilm vorhandenen oberflächenmodifizierenden Verbindungen beeinträchtigt ist. Die erfindungsgemäß verwendeten Fluortenside zeichnen sich dabei, im Gegensatz zu anderen bekannten Tensiden, durch eine verbesserte Bioverträglichkeit aus. Fluortenside vereinigen in sich eine im Vergleich zu nichtfluorhaltigen Emulgatoren verbesserte Bioverträglichkeit und außergewöhnliche Oberflächenaktivität.

Bevorzugt sind Fluortenside der allgemeinen Formel

R_{F}-Rₚₒₗ

, wobei R_{F} eine lineare oder verzweigte Perfluoralkylgruppe mit mehr als 5 Kohlenstoffatomen ist und Rₚₒₗ einen polaren Kohlenwasserstoffrest darstellt, der mindestens eine funktionelle Gruppe umfasst welche ausgewählt ist aus der Reihe CO-NH(R), CO-NH(R)₂, COO-, COOR, SO₃-, SO₂-N(R)₂, CH₂-O-R, PO₂H, PO₃H, wobei R ein Alkyl darstellt.

Bevorzugt sind ferner Fluortenside, deren Molmasse größer als 400 g/mol ist, deren Oberflächenspannung in wässriger Lösung kleiner als 30 mNm ist bzw. die mindestens 6 vollfluorierte Kohlenstoffatome aufweisen.

Die bevorzugte Konzentration des Fluortensids liegt unterhalb der kritischen Micellenbildungskonzentration, vorzugsweise kleiner als 0,1 % ist und die bevorzugte Konzentration des Fluorcarbons ist größer als 60 Gew.-%, vorzugsweise größer als 90 Gew.-%.

Darüberhinaus werden die schädigenden Wirkungen der Fluortenside in den erfindungsgemäßen Präparationen durch die Wirkungen der unpolaren Komponente noch weiter unterdrückt. Dies gelingt durch die Einbindung der Fluortenside in die unpolare Matrix, wodurch die Erniedrigung der Oberflächenspannung der wässrigen Bestandteile erreicht wird, ohne die Verhältnisse aufzuheben, die durch die natürlichen oberflächenmodifizierenden Verbindungen des Tränenfilms hervorgerufen werden. Dabei wird ausgenutzt, daß die Wechselwirkungen der Fluortenside mit den unpolaren Komponenten stärker ist als mit den lipophilen Bestandteilen der Augenoberfläche.

Auf diese Weise können die bekannten oberflächenaktiven Eigenschaften von Tensiden für extraokulare Anwendungen nutzbar gemacht werden. In Kombination mit den Eigenschaften von Wasser und der unpolaren Matrix bewirken die Fluortenside die filmbildenden Eigenschaften der erfindungsgemäßen Präparationen, und ermöglichen so eine gleichmäßige, dünnfilmige Benetzung der Hornhaut.

Als unpolare Komponenten eignen sich insbesondere Fluorcarbone oder Silikonöl. Werden Fluorcarbone als unpolare Komponente eingesetzt, können die bekannten vorteilhaften Eigenschaften dieser Substanzen, wie z. B. die hohe Sauerstofflöslichkeit und die Schmier- bzw. Schutzfunktion, ausgenutzt werden. Durch die sauerstofftransportierenden Eigenschaften der Fluorcarbone wird die Hornhaut ausreichend mit Sauerstoff versorgt, ohne daß der Visus durch ungleichmäßige Schichtdicken oder milchige Emulsionen beeinträchtig ist und ohne daß sich zwischen der hydrophoben Hornhaut und den oleophoben Fluorcarbonen Sperrschichten bilden, die den Sauerstoffübergang behindern. Hervorzuheben ist hierbei, daß das Fluorcarbon nicht eines Trägers bedarf (wie beispielsweise in der EP 089 815), sondern selbst als Träger für die Fluortenside wirkt.

Die Ausbildung von dünnen Filmen durch den Lidschlag und die Spreitung der nicht mischbaren Komponenten führt nicht nur zu guten Gleiteigenschaften des erfindungsgemäßen Tränenersatzmittels, sondern ist auch wegen der dadurch erfolgenden feinen Verteilung außerdem für eine verlängerte Verweildauer auf der Oberfläche verantwortlich. Unterstützt wird dies noch dadurch, daß die dünnen Filme spontan möglichst geringe Schichtdicken anstreben, die gewählten Oberflächen- und Grenzflächeneigenschaften gleichzeitig aber einem Aufreißen der Filme entgegenwirken. Durch geeignete Wahl der Komponenten des Tränenersatzmittels kann die Oberflächenspannung des Tränenersatzmittels und die Grenzflächenspannung zwischen Tränenersatzmittel und Augenoberfläche so abgestimmt werden, daß die Summe aus beiden Größen kleiner als die Oberflächenspannung der Augenoberfläche wird. Dies ermöglicht insbesondere eine verbesserte Filmbildung durch Spreiten auf der Augenoberfläche. Die durch Spreitung entstehenden filmartigen Schichtstrukturen können die Funktion des natürlichen Tränenfilms übernehmen, wobei die Hornhaut benetzt und geschützt wird, ohne daß die Hornhautoberfläche dazu hydrophilisiert werden muß.

Die Gleiteigenschaften und die Verweildauer können noch weiter verbessert werden, wenn das erfindungsgemäße Tränenersatzmittel zunächst in der Form eines Polyaphrongels vorliegt. Es wurde herausgefunden, daß sich derartige Gele aus den erfindungsgemäßen Komponenten unter der Einwirkung der beim Lidschlag auftretenden hohen Scherspannungen in den flüssigen Zustand zersetzen. Diese Zersetzung ist irreversibel, sobald die Polyaphronstruktur im gesamten Volumenbereich des Gels komplett zerstört ist. Es hat sich gezeigt, daß durch Scherspannungskräfte, die beispielsweise bei einem Lidschlag erzeugt werden, ein bestimmtes Volumen aus dem Gel durch irreversible Verflüssigung freigesetzt wird und der verflüssigte Anteil des Gels sich anschließend durch Spreitung der unmischbaren Komponenten als dünner Film gleichmäßig auf glatten Oberflächen verteilt. Vorteilhafterweise wird das gelförmige Tränenersatzmittel in den Bindehautsack eingebracht, wo es ein gelförmiges Reservoir bildet. Bei jedem Lidschlag wird jeweils ein Teil des Gels verflüssigt und anschließend als dünner Film über der Hornhautoberfläche fein und gleichmäßig verteilt.

Das Tränenersatzmittel weist vorzugsweise einen Brechungsindex von 1.334 bis 1.338 auf. Ferner bevorzugt ist dass die Summe aus Oberflächenspannung des Tränenersatzmittels und der Grenzflächenspannung zwischen Tränenersatzmittel und Augenoberfläche kleiner ist als die Oberflächenspannung der Augenoberfläche.

Mit den beschriebenen erfindungsgemäßen Präparationen gelingt es in vorzüglicher Weise die Anforderungen an Tränenersatzmittel zu erfüllen. Dabei ist besonders zu erwähnen, daß zwar die Einzelkomponenten selbst wichtige Funktionen beisteuern, es aber erst durch die Kombination der einzelnen Komponenten gelingt, die vorzüglichen Eigenschaften der Fluortenside und der unpolaren Komponente miteinander zu kombinieren.

Hierbei ist insbesondere die Kombination der sauerstofftransportierenden Eigenschaften und die Aufnahmefähigkeit für wasserlösliche Nährstoffe mit den Eigenschaften einer hohen Transparenz und guter Filmbildung von entscheidender Bedeutung. Neben der Übernahme von Funktionen wie Benetzung, Reibungsminderung, Nährstoff- und Sauerstoffversorgung können auch weitere günstige Eigenschaften der Präparationen genutzt werden, wie z. B. die oleophoben Eigenschaften der Präparationen, was dazu führt, daß Talgablagerungen verhindert werden und es nicht zu einem Verkleben des Lids kommt. Ein weiterer Vorteil ergibt sich aus den konservierenden Eigenschaften der unpolaren Komponenten, weil dadurch bedingt nicht alle Herstellungsschritte unter sterilen Bedingungen erfolgen müssen. Das heißt, die Präparationen können nach Fertigstellung z. B. durch Behandlung bei 121° C über 15 Minuten im Autoklaven sterilisiert werden, wobei auf zusätzliche Konservierungsmittel verzichtet werden kann.

Die folgenden Beispiele sollen die Herstellung und Verwendung der erfindungsgemäßen Präparate näher erläutern:

### Beispiel 1 (fällt nicht unter die Ansprüche):

Aus 0,1 g Fluowet® (Markenname, Firma Clariant) OTL, 0,9 g Wasser und 99 g Perfluorophenanthren (mit einer Oberflächenspannung von σ=19 mNm;) werden homogenisiert, bei 121° C 15 Minuten sterilisiert und anschließend mittels Spritze auf einen Spiegel aufgetragen und verteilt, wobei die Oberfläche durch einen gleichmäßigen, nicht aufreißenden Film benetzt wird.

### Beispiel 2 (fallt nicht unter die Ansprüche):

Aus dem Tetraethylpiperaziniumsalz der Perfluoroctansäure (σ=15,9 mNm; 0,1 g), balanced salt solution (BSS); 0,9 g und Perfluorophenanthren (σ=19 mNm; 99 g) bzw. Perfluoralkylethanol-Oxethylat (σ=19 mNm; 0,1 g) BSS (0,9 g) und Perfluorophenanthren (σ=19 mNm; 99 g) wurden nach bekannten Verfahren Polyaphrongele hergestellt und bei 121° C 15 Minuten im Autoklaven sterilisiert. Die gelartigen Präparate wurden in Spritzen abgefüllt und in einem Draize-Test untersucht. Im Draize-Test kam es zu keinen Reizungen. Auch zwei Wochen nach der Gel-Applikation zeigten die Kaninchen keine Beeinträchtigung der Tränensekretion, der Schleimproduktion oder der Sekretion der Lidranddrüsen. Auch nach Weiterführung der täglichen Behandlung über 16 Wochen ergaben sich keine Anzeichen für eine Unverträglichkeit.

### Beispiel 3 (fallt nicht unter die Ansprüche):

Eine nach Beispiel 2 hergestellte Präparation wird in das Auge von Kaninchen gegeben. Kurz danach erfolgt die Applikation eines bekannten Behandlungsmittels zur Erweiterung der Pupillen. Dabei wird festgestellt, daß das Mittel zur Erweiterung der Pupillen seine Wirkung verliert, sobald sich ein dünner Film des erfindungsgemäßen Tränenersatzmittels auf der Hornhaut gebildet hat. Dies dient als Nachweis zur Fähigkeit der erfindungsgemäßen Präparation, dünne, geschlossene Filme über der Hornhaut zu bilden, wie bei der natürlichen Träne. Darüberhinaus zeigt dieses Beispiel, daß die erfindungsgemäßen Präparationen eine Schutzfunktion ausüben, indem sie verhindern, daß wasserenthaltende oder wasserlösliche Substanzen mit der Augenoberfläche in Berührung kommen, sobald sie einen dünnen Film auf der Hornhaut ausgebildet haben.

### Beispiel 4:

Aus einer 10%igen Lösung der Perfluoroctansäure-tetraethylpiperaziniumsalz (mit einer Oberflächenspannung σ=15,9 mNm) in Wasser und hochgereinigtem Silikonöl 1000 mPas wird ein Gel durch Aufschäumen der wässrigen Lösung und Eintragen des Silikonöls erzeugt. Nach der Sterilisation bei bei 121° C für 15 Minuten wird diese Präparation auf eine wasserbeladene Oberfläche gegeben. Es setzt eine sponatane Spreitung ein.

## Patentansprüche

1. Verwendung eines Gels enthaltend mindestens ein wasserlösliches Fluortensid, Wasser und eine unpolare Komponente, ausgewählt aus teilfluorierten Fluorcarbonverbindungen der allgemeinen Formel R_{F}R_{H} oder R_{F}R_{H}R_{F}, sowie Fluorcarbon-Oligomeren des Typs (R_{F})ₓR_{H}, oder aus einer Mischung hiervon oder Silikonöl, zur Herstellung eines Tränenersatzmittels, wobei das Gel eine Polyaphronstruktur aufweist.

2. Verwendung nach einem der voranstehenden Ansprüche,
**dadurch gekennzeichnet, dass** das Fluortensid nach der allgemeinen Formel
R_{F}-Rₚₒₗ
dargestellt ist, wobei R_{F} eine lineare oder verzweigte Perfluoralkylgruppe mit mehr als 5 Kohlenstoffatomen ist und Rₚₒₗ einen polaren Kohlenwasserstoffrest darstellt, der mindestens eine funktionelle Gruppe umfasst welche ausgewählt ist aus der Reihe CO-NH(R), CO-NH(R)₂, COO-, COOR, SO₃-, SO₂-N(R)₂, CH₂-O-R, PO₂H, PO₃H, wobei R ein Alkyl darstellt.

3. Verwendung nach einem der voranstehenden Ansprüche, **dadurch gekennzeichnet, dass** die Molmasse des Fluortensids größer als 400 g/mol ist, die Oberflächenspannung der Fluortenside in wässriger Lösung kleiner als 30 mNm ist.

4. Verwendung nach einem der voranstehenden Ansprüche,
**dadurch gekennzeichnet, dass** die Konzentration des Fluortensids unterhalb der kritischen Micellenbildungskonzentration liegt, vorzugsweise kleiner als 0,1 % ist und die Konzentration des Fluorcarbons größer als 60 Gew.-%, vorzugsweise größer als 90 Gew.-% ist.

5. Verwendung nach einem der voranstehenden Ansprüche,
**dadurch gekennzeichnet, dass** das wasserlösliche Fluortensid mindestens 6 vollfluorierte Kohlenstoffatome aufweist.

6. Verwendung nach einem der voranstehenden Ansprüche,
**dadurch gekennzeichnet, dass** das Tränenersatzmittel einen Brechungsindex von 1.334 bis 1.338 aufweist.

7. Verwendung nach einem der voranstehenden Ansprüche,
**dadurch gekennzeichnet, dass** das Tränenersatzmittel auf der Hornhaut einen dünnen Film ausbildet.

8. Verwendung nach einem der voranstehenden Ansprüche,
**dadurch gekennzeichnet, dass** die Summe aus Oberflächenspannung des Tränenersatzmittels und der Grenzflächenspannung zwischen Tränenersatzmittel und Augenoberfläche kleiner ist als die Oberflächenspannung der Augenoberfläche.

9. Verwendung nach einem der voranstehenden Ansprüche,
**dadurch gekennzeichnet, dass** sich das Gel unter Einwirkung von Scherspannungskräften zumindest teilweise irreversibel verflüssigt.

10. Verwendung nach Anspruch 9,
**dadurch gekennzeichnet, dass** das Tränenersatzmittel in den Bindehautsack einbringbar ist, wobei das Tränenersatzmittel dort ein gelförmiges Reservoir ausbildet, von dem sich bei jedem Lidschlag ein Teil verflüssigt.

11. Verwendung nach Anspruch 10,
**dadurch gekennzeichnet, dass** der verflüssigte Teil des Tränenersatzmittels auf der Hornhaut einen dünnen Film ausbildet.

12. Verwendung nach einem der vorhergehenden Ansprüche,
**dadurch gekennzeichnet, dass** das Gel aus wasserlöslichem Fluortensid, Wasser und der unpolaren Komponente nach Fertigstellung sterilisiert wird.

## Claims

1. Use of a gel containing at least one water-soluble fluorinated surfactant, water and one homopolar component selected from among partially fluoridated fluorocarbon compounds of the general formula R_{F}R_{H} or R_{F}R_{H}R_{F}, as well as fluorocarbon oligomers of the type (R_{F})xR_{H}, or a mixture thereof, or silicon oil, for the manufacture of a tear substitution agent whereby the gel has a polyaphron structure.

2. Use in accordance with one of the preceding claims,
**characterised in that** the fluorinated surfactant is presented in accordance with the general formula
R_{F}-Rₚₒₗ
whereby R_{F} is a linear or branched perfluoroalkyd group having more than 5 carbon atoms and Rpol is a polar hydrogen residue comprising at least one functional group selected from the series CO-NH(R), CO-NH(R)₂, COO-, COOR, SO₃, SO₂-N(R)₂, CH₂-O-R, PO₂H, PO₃H whereby R is an alkyl.

3. Use in accordance with one of the preceding claims,
**characterised in that** the molecular mass of the fluorinated surfactant is greater than 400 g/mol, the surface tension of fluorinated surfactants in aqueous solution are less than 30 m/Nm.

4. Use in accordance with one of the preceding claims,
**characterised in that** the concentration of the fluorinated surfactant is below the critical micellae formation concentration, preferably lower than 0.1% and the concentration of fluorocarbons is greater than 60 weight percent, preferably greater than 90 weight percent.

5. Use in accordance with one of the preceding claims,
**characterised in that** the water-soluble fluorinated surfactant has at least 6 fully fluoridated carbon atoms.

6. Use in accordance with one of the preceding claims,
**characterised in that** the tear substitution agent has a refraction index of 1.334 to 1.338.

7. Use in accordance with one of the preceding claims,
**characterised in that** the tear substitution agent forms a thin film on the cornea.

8. Use in accordance with one of the preceding claims,
**characterised in that** the sum of the surface tension of the tear substitution agent and the interfacial tension between the tear substitution agent and the surface of the eye is smaller than the surface tension of the surface of the eye.

9. Use in accordance with one of the preceding claims,
**characterised in that** part of the gel liquefies irreversibly under the effect of shearing forces.

10. Use in accordance with claim 9,
**characterised in that** the tear substitution agent can be carried into the conjunctival sac, whereby the tear substitution agent forms a gel-like reservoir, part of which liquefies at every blink of the eye.

11. Use in accordance with claim 10,
**characterised in that** the liquefied part of the tear substitution agent forms a thin film on the cornea.

12. Use in accordance with one of the preceding claims,
**characterised in that** the gel of water-soluble fluorinated surfactant, water and the homopolar component is sterilised after manufacture.

## Revendications

1. Utilisation d'un gel contenant au moins un tensioactif fluoré soluble dans l'eau, de l'eau et un composant non polaire, choisi parmi des composés fluorocarbonés partiellement fluorés de formule générale R_{F}R_{H} ou R_{F}R_{H}R_{F}, ainsi que des oligomères fluorocarbonés du type (R_{F})ₓR_{H}, ou un mélange de ceux-ci ou une huile siliconée, pour la préparation de larmes artificielles, où le gel présente une structure polyaphronique.

2. Utilisation selon la revendication 1, **caractérisée en ce que** le tensioactif fluoré est représenté par la formule générale
R_{F} - Rₚₒₗ
dans laquelle R_{F} est un groupe perfluoroalkyle linéaire ou ramifié comportant plus de 5 atomes de carbone et Rₚₒₗ est un reste hydrocarboné polaire qui comprend au moins un groupe fonctionnel choisi dans la série formée par CO-NH(R), CO-NH(R)₂, COO-, COOR, SO₃-, SO₂-N(R)₂, CH₂-O-R, PO₂H, PO₃H, où R représente un radical alkyle.

3. Utilisation selon l'une quelconque des revendications qui précèdent, **caractérisée en ce que** la masse molaire du tensioactif fluoré est supérieure à 400 g/mole, et que la tension superficielle du tensioactif fluoré en solution aqueuse est inférieure à 30 mNm.

4. Utilisation selon l'une quelconque des revendications qui précèdent, **caractérisée en ce que** la concentration du tensioactif se situe au-dessous de la concentration critique de formation de micelles, et est de préférence inférieure à 0,1%, et la concentration de fluorocarbone est supérieure à 60% en poids, de préférence supérieure à 90% en poids.

5. Utilisation selon l'une quelconque des revendications qui précèdent, **caractérisée en ce que** le tensioactif soluble dans l'eau présente au moins 6 atomes de carbone totalement fluorés.

6. Utilisation selon l'une quelconque des revendications qui précèdent, **caractérisée en ce que** les larmes artificielles présentent un indice de réfraction de 1.334 à 1.338.

7. Utilisation selon l'une quelconque des revendications qui précèdent, **caractérisée en ce que** les larmes artificielles forment un film mince sur la cornée.

8. Utilisation selon l'une quelconque des revendications qui précèdent, **caractérisée en ce que** la somme de la tension superficielle des larmes artificielles et de la tension d'interface entre les larmes artificielles et la surface de l'oeil est inférieure à la tension superficielle de la surface de l'oeil.

9. Utilisation selon l'une quelconque des revendications qui précèdent, **caractérisée en ce que** le gel se fluidifie de manière au moins partiellement irréversible sous l'action de forces de cisaillement.

10. Utilisation selon la revendication 9, **caractérisée en ce que** les larmes artificielles peuvent être appliquées dans le sac conjonctif, les larmes artificielles y formant un réservoir de gel dont une partie se fluidifie à chaque battement de paupières.

11. Utilisation selon la revendication 10, **caractérisée en ce que** la partie fluidifiée des larmes artificielles forme un film mince sur la cornée.

12. Utilisation selon l'une quelconque des revendications qui précèdent, **caractérisée en ce que** le gel contenant un tensioactif soluble dans l'eau, de l'eau et le composant non polaire est stérilisé après sa fabrication.
